# EUROPEAN PATENT APPLICATION

(11) **EP 4 455 293 A1**
(43) Date of publication of application: **30.10.2024**
(21) Application number: 23315106.7
(22) Date of filing: 25.04.2023
(51) Int. Cl.: C12N 15/82, C12N 9/22

(54) **METHODS FOR PROVIDING A PLANT HAVING INCREASED RESISTANCE TO A VIRUS OF THE LUTEOVIRIDAE FAMILY**

(71) Applicant: Limagrain Europe, 63360 Saint-Beauzire (FR)
(72) Inventor: BERRY, Simon Timothy, Litcham, Norfolk, PE32 2QQ (GB); SALLAUD, Christophe, 63110 Beaumont (FR); LAFARGE, Stephane, 63670 La Roche Blanche (FR); THROUDE, Mickael, 63116 Beauregard L'Evêque (FR); BERTIN, Benjamin, 03300 Creuzier Le Vieux (FR); SPECEL, Sébastien, 63260 Montpensier (FR); COMADRAN, Jordi, 63200 Riom (FR)
(74) Representative: Lavoix

(57) **Abstract**

The present invention concerns methods for providing a plant having increased resistance to a virus of the *Luteoviridae* family, in particular to Barley Yellow Dwarf Virus, and plants having increased resistance to a virus of the *Luteoviridae* family

## Description

The present invention concerns methods for providing a plant having increased resistance to a virus of the *Luteoviridae* family, in particular to Barley Yellow Dwarf Virus, and plants having increased resistance to a virus of the *Luteoviridae* family.

### BACKGROUND OF THE INVENTION

Barley Yellow Dwarf Virus (BYDV), caused by virus species in the *Luteoviridae* family and vectored by aphids, is the most widely distributed viral disease of cereal crops. BYDV affects more than 150 species within the *Poaceae* family, including barley (*Hordeum vulgare*), bread wheat (*Triticum aestivum*) and maize *(Zea mays).* Due to the broad host range of BYDV and the widespread distribution of aphid vectors, BYDV is an economically important disease in all cereal growing regions of the world, including Europe, Asia, Africa, and North/South America. BYDV infected plants display stunted growth, leaf chlorosis, delayed flowering and sterility, symptoms which ultimately reduce both grain yield and quality (Walls et al. 2019, Agric. 9:1-16). Losses attributed to BYDV in wheat and barley average 30% but can reach up to 80%, with losses most severe when cereals are infected at the seedling stage. In the UK, a 30% average yield loss in wheat and barley production would equate to losses of 4.5 million tonnes and 2.6 million tonnes, respectively, based on the UK wheat and barley production 2015-2019 five-year averages.

BYDV is caused by several species of positive-sense, single-stranded RNA (ssRNA) viruses from the 'barley and cereal yellow dwarf virus' complex. BYDV viruses are 25 - 30 nm in diameter and icosahedral in shape (ICTV, 2011). BYDV viruses belong to the *Luteoviridae* family and are further classified by the International Committee on the Taxonomy of Viruses (ICTV) as belonging to either the *Luteovirus* genus, the *Polerovirus* genus or a currently 'unassigned' group (King et al. 2018, Arch. Virol. 163: 2601-2631). Species assigned to the *Luteovirus* genus include BYDV-PAV, which is the most prevalent BYDV species worldwide, BYDV-MAV, BYDV-PAS, BYDV-GAV, BYDV-kerll and BYDV-kerlll. The cereal yellow dwarf virus (CYDV) species CYDV-RPV and CYDV-RPS are found within the *Polerovirus* genus. At present, BYDV-RMV, BYDV-SGV and BYDV-GPV are unassigned to a specific genus (King et al. 2018, Arch. Virol. 163: 2601-2631). At least 25 aphid species are thought to transmit BYDV, with differing species acting as the principal vectors of specific BYDV strains (Adhikari et al. 2020, Crop Prot. 134:105171). The bird cherry-oat aphid (*Rhopalosiphum padi*) and the English grain aphid (*Sitobion avenae*), which are the most prevalent species in Europe and North America, are the principal vectors of BYDV-PAV and BYDV-MAV whilst *R. padi* and *Schizaphis graminum* are the principal vectors of CYDV-RPV and BYDV-GPV (Fabre et al. 2003, Phytopathology 93:1217-1222).

BYDV control methods are primarily vector targeted and include for instance delaying the sowing of autumn cereals when aphid numbers have declined, or the use of biological or chemical control methods.

Genetic resistance to BYDV can be conferred against the aphid vector or against viral species within the BYDV complex.

Host plant resistance to insect vectors can be described using the following categories: antixenosis (the non-preference for a plant host), antibiosis (resistance which has a negative effect on insect fecundity or survival) and tolerance (ability of a host to withstand or compensate for insect damage, compared to the damage caused on a susceptible host under similar insect pressure) (War et al. 2012, Plant Signal. Behav. 7:1306; Koch et al. 2016, Plant Sci. 7:1363). Many sources of genetic loci associated with antibiosis, antixenosis and tolerance to aphids in cereals have been identified from wild relatives such as *Aegilops tauschii, Triticum dicoccum* and *Hordeum vulgare* ssp. *Spontaneum* (Arradotir and Crespa-Herrera, 2021, Curr. Opin. Insect Sci. 45:59-68).

BYDV viral resistance can be categorised as either true resistance or tolerance. Resistance is defined as a significant reduction in the multiplication of the virus (viral titre) within the plant host, whilst tolerance minimises the symptoms or yield losses and has a limited effect on viral multiplication/spread. Viral resistance to BYDV has been most widely studied in barley and wheat. In barley, four named BYDV resistance/tolerance genes have been identified.

The semi-dominant gene *Ryd2* (*Yd2*) has been the most extensively utilised gene for BYDV resistance to date in barley breeding programs and was originally identified in Ethiopian spring barley landraces (Rasmusson and Schaller, 1959, Agron. J 51:661-664). *Ryd2* is found close to the centromere on the long arm of chromosome 3H and a number of genetic markers have been developed to track this gene, including the PCR-based markers *Ylp* and *YLM* (Ford et al. 1998, Mol. Breed. 4:23-31; Paltridge et al. 1998, Theor. Appl. Genet. 96:1170-117). The gene has been used to provide BYDV tolerance in a number of barley cultivars including Atlas68, Vixen, Coracle and the malting barley Franklin (Jarošová et al. 2016, Field Crop Res. 198:200-214). *Ryd2* reduces the viral titre of strains BYDV-PAV and BYDV-MAV in young plants but has varying efficacy in mature plants and may not provide tolerance to all CYDV-RPV isolates (Riedel et al. 2011, Theor. Appl. Genet. 123:69-76). *Ryd2* is thought to act by limiting replication of the virus within the plant phloem but does not prevent movement of the virus within the infected host plant (King et al. 2002, Arch. Virol. 163: 2601-2631).

Of the remaining BYDV resistance genes, the recessive tolerance gene *ryd1* was the first to be identified but has not been mapped or used in breeding programs due to the limited nature of its efficacy (Rasmusson and Schaller, 1959, Agron. J 51:661-664). *Ryd3,* on the short arm of 6H near the centromere, was identified in an Ethiopian landrace and confers tolerance to BYDV-MAV and BYDV-PAV (Niks et al. 2004). Microsatellite markers, such as *HVM74,* have been developed to screen for *Ryd3* (Niks et al. 2004, Theor. Appl. Genet. 109: 1536-1543). Pyramiding both *Ryd2* and *Ryd3* in barley doubled haploid lines confers quantitative resistance against BYDV-PAV Viruliferous aphids (Riedel et al. 2011, Theor. Appl. Genet. 123:69-76). The gene *Ryd4^{Hb}* was identified in *H. bulbosum* and maps to the long arm of 3H in barley using the CAPS marker *TC134544* (Scholz et al. 2009, Theor. Appl. Genet. 119:837-849). A lack of BYDV symptoms and low ELISA values were observed in *Ryd4^{Hb}* carrying lines that were infected with BYDV-PAV, suggesting a true form of resistance to BYDV (Scholz et al. 2009, Theor. Appl. Genet. 119:837-849). However, a subsequent aphid feeding study demonstrated that the *Ryd4^{Hb} H. bulbosum* donor line A17 could not be infected with *R. padi* and *S.* avenae carrying BYDV-PAV, BYDV-MAV or CYDV-RPV (Schliephake et al. 2013, Entomol. Exp. Appl. 146:347-356). *R. padi* also showed reduced phloem feeding and the development of fewer nymphs on the A17 line when compared to a susceptible barley control (Schliephake et al. 2013, Entomol. Exp. Appl. 146:347-356). This therefore suggests that *Ryd4^{Hb}* provides resistance to the aphid vector and not to isolates within the BYDV complex.

The causal genes underlying BYDV resistance in barley are unknown.

In wheat, four named BYDV genes have been identified, however the underlying genes and mechanisms of resistance/tolerance remain unknown. *Bdv1,* identified in Brazilian wheat, provides tolerance to BYDV-MAV isolates under field conditions (Ginkel and Van Henry, 2002, Breeding for BYDV tolerance/resistance in CIMMYT bread wheats targeted to developing countries. In: Barley yellow dwarf disease: recent advances and future strategies. CIMMYT, Mexico City: 82-92). *Bdv1* is linked to the genes *Lr34* and *Yr18* on 7D that provide resistance to leaf rust and yellow rust, respectively, at the adult plant stage (Singh, 1993, Plant Dis. 77:1103). *Bdv2, Bdv3* and *Bdv4* originate from the wild wheat relative *Thinopyrum intermedium. Bdv2* confers resistance to BYDV-PAV, BYDV-GAV and BYDV-GPV isolates by slowing disease development (Jarošová et al. 2016, Field Crop Res. 198:200-214), whilst *Bdv3* confers complete resistance to CYDV-RPV, by limiting CYDV movement in sieve cell plates, and moderate resistance to BYDV-PAV and BYDV-MAV (Zhang et al. 2009, J. Genet. Genomics 36:567-573). Both *Bdv2* and *Bdv3* have been introgressed onto chromosome 7D through wheat-*Th*. *intermedium* translocations. *Bdv4,* introgressed on 2D, provides resistance BYDV-GPV, BYDV-GAV and the Chinese isolate PAGV (Lin et al. 2007, Euphytica 158:109-118).

Studies have also identified QTL associated with BYDV resistance. QTL on chromosomes 2H, 5H and 7H in barley (Riedel et al. 2011, Theor. Appl. Genet. 123:69-76; Hu et al. 2019, Sci. Rep. 9:1-10) and chromosomes 2A, 2B, 5A, 6A and 7A in wheat (Choudhury et al. 2019, Plant Dis. 103:2798-2803, Choudhury et al. 2019, BMC Genomics 20) have been detected, however these loci have not been extensively analysed. Transgenic approaches have also been shown to provide resistance to BYDV, however work is still being undertaken to improve the efficacy of transgenic methods. McGrath et al. (1997) transformed the barley cultivar Golden Promise with the coat protein of BYDV-PAV and challenged the recovered transformants with viruliferous aphids. Moderate resistance was achieved, but not all transformants carrying the coat protein transgene showed resistance (McGrath et al. 1997, Eur. J Plant Pathol. 103:695-710). Similarly, transformation of Golden Promise with a BYDV-PAV hairpin RNA (hpRNA) transgene provided high levels resistance in nine out of 25 transformed barley lines in a study by Wang et al. (2000, Mol. Plant Pathol. 1:347-356).

A recent study by Alquicer et al. (2023, Viruses 15:716-735) looking at the expression analysis of nine classes of known disease resistance genes (NBS-LRR, CC-NB-LRR class, RLK, casein kinase, protein kinase genes, protein phosphatase subunits and the transcription factors (TF), MYB superfamily, GRAS and MADS-box TF), in both tolerant/resistant and susceptible lines of wheat and barley, failed to identify any candidate genes responsible for resistance to BYDV. There is still a need to identify BYDV resistance genes in barley, wheat and other plant species.

### SUMMARY

The invention relates to a method for providing a plant having increased resistance to a virus of the *Luteoviridae* family, which comprises modifying the plant so that it is able to express a vacuolar-type ^{H+}-ATPase subunit E (VHA-E), or an ortholog thereof, having disrupted interaction with the virus of the *Luteoviridae* family.

The invention also relates to a method for providing a plant having increased resistance to a virus of the *Luteoviridae* family, which comprises mutating a gene encoding vacuolar-type H+-ATPase subunit E (VHA-E) in the genome of the plant to encode a p.H160P VHA-E protein, wherein the plant is barley (Hordeum vulgare L.), common wheat (Triticum aestivum L.), durum wheat *(Triticum durum* or *Triticum turgidum* subsp. *Durum),* maize (Zea mays), or rice (Oryza sativa), or to encode an ortholog of p.H160P VHA-E protein when the plant is from a different plant species.

Also provided is a method for providing a plant having increased resistance to a virus of the *Luteoviridae* family, which comprises inhibiting expression of vacuolar-type ^{H+}-ATPase subunit E (VHA-E) protein, or an ortholog thereof, in the plant.

The invention further relates to genetically modified plant having increased resistance to a virus of the *Luteoviridae* family, which has been obtained by a method according to the invention.

In another aspect, the invention relates to a method for identifying mutant genes encoding vacuolar-type H⁺-ATPase subunit E (VHA-E) protein, or an ortholog thereof, conferring increased resistance to a virus of the *Luteoviridae* family to a plant susceptible to the virus of the *Luteoviridae* family, which comprises:
a) screening a genomic DNA library of said plant for identifying point mutation(s) in a gene encoding VHA-E protein, or an ortholog thereof;
b) generating genetically modified plants comprising a point mutation in a gene encoding VHA-E protein, or an ortholog thereof, as identified at step a);
c) characterizing if said genetically modified plants have increased resistance to the virus of the *Luteoviridae* family, or not, compared to plants susceptible to the virus of the *Luteoviridae* family comprising non mutated genes encoding VHA-E protein, or an ortholog thereof, and;
d) identifying the mutant gene(s) encoding VHA-E protein, or an ortholog thereof, that are present in said genetically modified plants having increased resistance to the virus of the *Luteoviridae* family, as conferring increased resistance to the virus of the *Luteoviridae* family.

In still another aspect, the invention relates to a method for identifying mutant plants having resistance to a virus of the *Luteoviridae* family, which comprises:
a) screening a plant library for identifying, in one or more plants of the library, point mutation(s) in a gene encoding VHA-E protein, or an ortholog thereof;
b) characterizing if said plants identified with point mutation(s) in a gene encoding VHA-E protein, or an ortholog thereof, have increased resistance to the virus of the *Luteoviridae* family, or not, compared to plants susceptible to the virus of the *Luteoviridae* family comprising non mutated genes encoding VHA-E protein, or an ortholog thereof.

### DETAILED DESCRIPTION

The inventors have identified that the *Yd2* variant causal for BYDV resistance in barley is the gene encoding a V-type proton ATPase subunit E (VHA-E) (Horvu_MOREX_3H01G350400). BYDV resistance is conferred by a missense mutation A/C in *VHA-E exon* 4, resulting in a Histidine to Proline mutation at amino acid position 160 of the encoded polypeptide.

The inventors further elucidated that BYDV MP and RTD proteins interact with VHA-E protein of wheat and barley. It is thus hypothesized that BYDV uses VHA-E to perform its infection, and that this interaction is altered in BYDV resistant plants, in light of the inability of the virus to infect plants with high expression of the resistant allele (variant) of *VHA-E.*

Sequences homologous to barley *VHA-E* gene were identified in wheat (three homeolog sequences), in maize (two homeolog sequences), and in rice (one sequence).

Viruses of the same family have often similar infection mechanisms. Accordingly, the VHA-E protein which is the molecular target of BYDV in wheat and barley may also be the target of another virus of the *Luteoviridae* family in other plant species. It was recently shown that *Barley stripe mosaic virus* (BSMV) replicase γa interacts with V-ATPase catalytic subunit B2 (VHA-B2), leading to disruption of the interaction between VHA-B2 and VHA-E, which impairs the membrane localization of VHA-B2 and suppresses V-ATPase activity. Suppression of V-ATPase activity results in impaired vacuolar acidification, which is essential for plant antiviral immunity (Yang et al., The EMBO Journal (2022) 41 :e108713).

The invention relates to a method for providing a plant having increased resistance to a virus of the *Luteoviridae* family, which comprises modifying the plant so that it can express a vacuolar-type H⁺-ATPase subunit E (VHA-E), or an ortholog thereof, having disrupted interaction with the virus of the *Luteoviridae* family. The method may comprise, in particular, the mutation of a gene encoding VHA-E, or an ortholog thereof, in the genome of the plant to encode a VHA-E protein, or an ortholog thereof, having disrupted interaction with the virus of the *Luteoviridae* family. The method may also comprise introducing into the plant a nucleic acid encoding a VHA-E protein, or an ortholog thereof, having disrupted the interaction with the virus of the *Luteoviridae* family.

The invention also relates to a method for providing a plant having increased resistance to a virus of the *Luteoviridae* family, which comprises mutating a gene encoding VHA-E in the genome of the plant to encode a p.H160P VHA-E protein when the plant is barley *(Hordeum vulgare L.),* wheat, in particular common wheat *(Triticum aestivum L.)* or durum wheat *(Triticum durum* or *Triticum turgidum* subsp. *Durum),* maize *(Zea mays),* or rice *(Oryza sativa),* or an ortholog of p.H160P VHA-E protein when the plant is from a different plant species.

According to another alternative, the invention relates to a method for providing a plant having increased resistance to a virus of the *Luteoviridae* family, which comprises inhibiting expression of vacuolar-type H⁺-ATPase subunit E (VHA-E) protein, or an ortholog thereof, in the plant. According to some embodiments, the method comprises knocking-down a gene encoding VHA-E, or an ortholog thereof.

According to some embodiments, the virus of the *Luteoviridae* family is a virus of the *Luteovirus* genus, the *Polerovirus* genus, or another genus group.

According to some embodiments, the virus of the *Luteoviridae* family is a Barley Yellow Dwarf Virus (BYDV). The virus of the *Luteoviridae* family is for instance a BYDV of one of the following species: BYDV-MAV, BYDV-PAS, BYDV-GAV, BYDV-kerll, BYDV-kerlll, BYDV-RMV, BYDV-SGV and BYDV-GPV.

According to some embodiments, the virus of the *Luteoviridae* family is a Cereal Yellow Dwarf Virus (CYDV). The virus of the *Luteoviridae* family is in particular a CYDV species of one of the following species: CYDV-RPV and CYDV-RPS.

According to some embodiments, the virus of the *Luteoviridae* family is a virus of the genus *Polerovirus,* such as Turnip yellows virus (TuYV), Maize Yellow Mosaic Virus (MYMV), Beet mild Yellowing virus (BMYV), or Potato Leafroll virus (PLRV).

According to some embodiments, the plant to which increased resistance to a virus of the *Luteoviridae* family is to be conferred is a cereal crop, in particular a plant species of the *Poaceae* family including barley (*Hordeum vulgare L.*)*,* wheat, in particular common wheat (*Triticum aestivum L.*) or durum wheat (*Triticum durum* or *Triticum turgidum* subsp. *Durum),* maize (*Zea mays*)*,* rice (*Oryza sativa*)*,* or rapeseed (*Brassica napus* L.), or beet or potato. Preferably, the plant is barley, common wheat, maize, or rice.

As used herein, "resistance" denotes the host's ability to limit pathogen multiplication, by contrast to "tolerance" that denotes the host's ability to reduce the effect of infection on its fitness, regardless of the level of pathogen multiplication. The resistance may be partial or complete.

In some embodiments, the plant is initially susceptible to infection with the virus of the *Luteoviridae* family; in these embodiments, partial or complete resistance is conferred by the methods disclosed herein. In some embodiments, the plant is initially partially resistant to infection with the virus of the *Luteoviridae* family; in these embodiments, partial resistance is increased, or complete resistance is conferred, by the methods disclosed herein.

As used herein, *VHA-E* denotes the gene encoding V-type proton ATPase subunit E (VHA-E) protein, a subunit of the V1 complex of vacuolar(H+)-ATPase (V-ATPase), a multi-subunit enzyme composed of a peripheral complex (V1) that hydrolyzes ATP and a membrane integral complex (V0) that translocates protons.

*VHA-E gene* in barley *(Hordeum vulgare L.)* comprises or consists of sequence SEQ ID NO: 1 (Horvu_MOREX_3H01G350400 chr3H:411658438..411661959). The CDS comprises or consists of sequence SEQ ID NO: 2 (Horvu_MOREX_3H01G350400_-_CDS chr3H:411658438..411661959) that encodes a polypeptide comprising or consisting of SEQ ID NO: 3 (Horvu_MOREX_3H01G350400_Translation chr3H:411658438..411661959).

In common wheat (*Triticum aestivum L*.):
- On chromosome 3A, *VHA-E* CDS sequence comprises or consists of SEQ ID NO: 4 (Gene-A:TraesCS3A01G238700_CDS chr3A:446473885..446480933) that encodes a polypeptide comprising or consisting of SEQ ID NO: 5 (Gene-A:TraesCS3A01G238700_CDS_translation chr3A:446473885..446480933);
- On chromosome 3B, *VHA-E* CDS sequence comprises or consists of SEQ ID NO: 6 (Gene-B:TraesCS3B02G267400_CDS chr3B:429271152..429276269) and encodes a polypeptide comprising or consisting of SEQ ID NO: 7 (Gene-B:TraesCS3B02G267400_CDS_translation chr3B:429271152..429276269);
- On chromosome 3D, *VHA-E* CDS sequence comprises or consists of SEQ ID NO: 8 (Gene-D:TraesCS3D02G239100_CDS chr3D:330636892..330643079) and encodes a polypeptide comprising or consisting of SEQ ID NO: 9 (Gene-D:TraesCS3D02G239100_CDS_transtation chr3D:330636892..330643079).

*VHA-E* gene in rice (*Oryza sativa*) comprises or consists of sequence SEQ ID NO: 10 (Os01g0659200 class=Sequence position=chr01:26811872..26816519) The CDS comprises or consists of sequence SEQ ID NO: 11 (Os01t0659200-01_CDS cds chromosome:IRGSP-1.0:1:26811872:26816519:1 gene) that encodes a polypeptide comprising or consisting of SEQ ID NO: 12 (Os01t0659200-01_CDS_translation cds chromosome:IRGSP-1.0:1:26811872:26816519:1 gene). An isoform of rice *VHA-E* CDS comprises or consists of sequence SEQ ID NO: 13 (Os01t0659200-01-cDNA Subunit E isoform 1 of vacuolar H+-ATPase).

*VHA-E* genes in maize *(Zea mays)* comprises or consists of 2 sequences SEQ ID NO: 14 (Zm00001d011504 8:152696094..152705701) and SEQ ID NO: 15 (Zm00001d043991_ReverseCom_sequence/!\ 3:215827891..215837507). The CDS comprises or consists of sequence SEQ ID NO: 16 (Zm00001d011504_-_CDS 8:152696094..152705701) that encodes a polypeptide comprising or consisting of SEQ ID NO: 17 (Zm00001 d01 1504_-_CDS_translation 8:152696094..152705701); or comprises or consists of sequence SEQ ID NO: 18 (Zm00001d043991_-_CDS 3:215827891..215837507) that encodes a polypeptide comprising or consisting of SEQ ID NO: 19 (Zm00001d043991_-_CDS_translation 3:215827891..215837507).

*VHA-E* orthologous genes and encoded proteins (i.e. homologs in different species that fulfill the same biological function) in other plants can readily be identified by the skilled person by available methods.

The methods for providing a plant having increased resistance to a virus of the *Luteoviridae* family may comprise mutating a gene encoding vacuolar-type H⁺-ATPase subunit E (VHA-E), or an ortholog thereof, in the genome of the plant.

In some embodiments, the mutation in the gene encoding VHA-E protein produces a VHA-E protein having disrupted interaction with the virus of the *Luteoviridae* family, thereby conferring a plant having increased resistance to a virus of the *Luteoviridae* family. In particular, when the virus is BYDV, the mutation in the gene encoding VHA-E protein produces a VHA-E protein having disrupted interaction with BYDV MP protein (SEQ ID NO: 20) and/or BYDV RTD protein (SEQ ID NO: 21). Determining if a mutation in the gene encoding VHA-E protein produces a VHA-E protein having disrupted interaction can readily be assayed by the skilled person, for instance by performing yeast two-hybrid experiments, such as described in example 5.

In some embodiments, the method comprises mutating the gene encoding VHA-E in a codon encoding Histidine at position 160 of the VHA-E protein, in particular when the plant is barley, common wheat, maize, or rice. In a different plant species, the method comprises mutating an ortholog of the gene encoding VHA-E protein in a codon homologous to the one encoding Histidine at position 160 of VHA-E protein of barley, common wheat, maize, or rice.

Preferably, the method comprises mutating the gene encoding VHA-E protein to encode a VHA-E protein having a histidine substituted for proline at position 160 of VHA-E protein (denoted as "p.H160P VHA-E protein"), in particular when the plant is barley, common wheat, maize, or rice.

In some embodiments, the method comprises mutating a gene encoding VHA-E protein in the genome of the plant to encode a p.H160P VHA-E protein when the plant is barley (*Hordeum vulgare L.*)*,* wheat, in particular common wheat (*Triticum aestivum L.*) or durum wheat (*Triticum durum* or *Triticum turgidum* subsp. *Durum*)*,* maize (*Zea mays*), or rice (*Oryza sativa*), or to encode an ortholog of p.H160P VHA-E protein when the plant is from a different plant species (e.g. rapeseed, beet or patato).

Depending on the ploidy of the plant to which resistance to a virus of the *Luteoviridae* family is to be conferred, the methods may comprise mutating at least one, e.g. one, two or three, homeologous gene(s) encoding VHA-E protein (e.g. in barley, wheat, in particular common or durum wheat, maize, or rice), or encoding an ortholog of VHA-E protein (e.g. in a plant species other than barley, wheat, maize, and rice).

The *Yd2* locus causal for viral resistance is known to be semi-dominant (Schaller 1964, 1984). Accordingly, even in the heterozygous state, the mutation of the *VHA-E* gene (or of an ortholog thereof) will confer an increased resistance to the virus of the *Luteoviridae* family. However, stronger resistance is expected to be conferred when the mutated *VHA-E* gene (or an ortholog thereof) is in the homozygous state.

In certain embodiments, the methods comprise mutating the gene encoding VHA-E protein at the heterozygous state. In certain embodiments, the methods comprise mutating the gene encoding VHA-E protein at the homozygous state.

In particular, when the plant is common wheat, the methods may comprise mutating one, two or three homeologous genes encoding VHA-E protein, at the heterozygous or homozygous state.

Mutating the gene encoding VHAE protein, or an ortholog therefor, may be performed by any suitable method known by the skilled person, such as a gene editing method.

The terms "gene editing" covers targeted mutations, these mutations can be random or directed mutations. By "targeted" it is intended that the localization of the mutation is chosen. In particular, by homologous recombination systems, a nucleotide exchange can be done to induce targeted mutations. Site-specific recombination systems have been described, for example, in international patent applications published as WO99/25821, WO99/25854, WO99/25840, WO99/25855, and WO99/25853. Briefly, a polynucleotide encoding a VHA-E protein having disrupted interaction with the virus of the *Luteoviridae* family, in particular a polynucleotide comprising a sequence encoding a VHA-E protein comprising a proline, instead of histidine, at amino acid position 160 (in particular in barley, wheat (in particular common or durum wheat), maize, or rice VHA-E protein), or an ortholog thereof, can be contained in a transfer cassette flanked by two non-identical recombination sites. An appropriate recombinase is needed for the integration of the transfer cassette fragment at the target site.

Other methods for "gene editing" include the use of a Double Strand Break (DSB), which enhances the probability of homologous recombination at a given position, and a repair template to induce a specific nucleotide exchange during DNA repair. Said methods have been improved by the development of Meganucleases, Zn finger nuclease and other tools like TALENs and CRISPR/Cas9 system that have been developed to obtain targeted DSB. The CRISPR/Cas9 system is one of the specific methods of "gene editing" whereas the Cas9 protein and an ARN are used for obtaining a targeted DSB. Alternatively, a simple DSB (double strand break) without repair template can be done on a targeted sequence to induce random mutations at this site. These mutations should be short insertions or deletions based on NHEJ (near Homologous End Joining) or MMEJ (microhomology mediated end joining).

Base editing is an alternative method for gene editing which is regarded as a more efficient approach than the CRISPR/Cas9 system. It relies on nucleotide conversions without the formation of DNA DSBs, with use of a tethered deaminase domain or nickase Cas9 for base conversion from A > G or C > T, and enables creating both single and multiple nucleotide modifications in cells (Azameti and Dauda, Front. Plant Sci., 2021, 12).

Furthermore an alternative gene editing method that does not require DSBs is prime editing. Prime editing uses a fusion protein, consisting of a catalytically impaired Gas 9 endonuclease fused to an engineered reverse transcriptase enzyme, and a prime editing guide RNA (pegRNA), capable of identifying the target site and providing the new genetic information to replace the target DNA nucleotides by targeted insertions, deletions, and base-to-base conversions without the need for double stra breaks (DSBs) or donor DNA templates.

The method for providing a plant having increased resistance to a virus of the *Luteoviridae* family may also comprise introducing into the plant a nucleic acid encoding a VHA-E protein, or an ortholog thereof, having disrupted interaction with the virus of the *Luteoviridae* family, thereby conferring a plant having increased resistance to a virus of the *Luteoviridae* family.

The nucleic acid may encode a VHA-E protein, or a functional ortholog thereof.

In some embodiments, the method comprises introducing into the plant a nucleic acid encoding a VHA-E protein, wherein the nucleic acid comprises a mutation in the codon encoding Histidine at position 160 of the VHA-E protein, in particular when the plant is barley, wheat (in particular common or durum wheat), maize, or rice. In a different plant species, the method introducing a nucleic acid encoding an ortholog of VHA-E protein, wherein the nucleic acid comprises a mutation in a codon homologous to the one encoding Histidine at position 160 of VHA-E protein of barley, wheat, maize, or rice.

Preferably, the method comprises introducing into the plant a nucleic acid encoding a VHA-E protein having a histidine substituted for proline at position 160 of VHA-E protein (p.H160P VHA-E protein), in particular when the plant is barley, wheat, maize, or rice.

The method for providing a plant having increased resistance to a virus of the *Luteoviridae* family may also comprise inhibiting expression of vacuolar-type H⁺-ATPase subunit E (VHA-E) protein, or an ortholog thereof, in the plant.

RNA interference (RNAi) can be induced in the plant to repress expression of *VHA-E* gene, or an ortholog thereof. By "RNA interference" means the biological process in which RNA molecules inhibit gene expression, typically endogenous gene expression by causing the destruction of specific mRNA molecules. According to an embodiment, the method for providing a plant having increased resistance to a virus of the *Luteoviridae* family thus comprises introducing into the plant a nucleic acid that inhibits *VHA-E* gene expression, or an ortholog thereof, having disrupted interaction with the virus of the *Luteoviridae* family. According to this aspect, a plant is transformed with a DNA or RNA molecule interfering with expression of *VHA-E* gene, or an ortholog thereof.

The RNA molecule can be a small interfering RNA (siRNA) molecule comprising 18-25 nucleotides of *VHA-E* gene, or an ortholog thereof. A siRNA typically comprises 18-25, e.g. 19-23 or preferably 19-21 contiguous nucleotides of the target gene. The RNA molecule can be a double stranded RNA (dsRNA). As used herein, a dsRNA molecule is a RNA molecule comprising at least one annealed, double stranded region. In certain aspects, the double stranded region comprises two separate RNA strands annealed together. In certain aspects, the double stranded region comprises one RNA strand annealed to itself, for example, as can be formed when a single RNA strand contains an inversely repeated sequences with a spacer in between ("hairpin RNA"). Expression of hairpin RNA in cells is typically accomplished by delivery of a DNA construct by plasmids or through viral or bacterial vectors, which is transcribed in a hairpin RNA.

A dsRNA molecule comprises a nucleic acid sequence complementary to about 21 to 2000 contiguous nucleotides of a target gene sequence discloses anywhere herein. For example, in certain aspects, an dsRNA molecule comprises a nucleic acid sequence complementary to about any of 21, 22, 23, 24, 25, 30, 100, 200, 300, 400, 500, 1000 to about any of 23, 24, 25, 30, 40, 50, 100, 200, 300, 400, 500, 1000, or 2000 contiguous nucleotides of a target gene sequence. For example, in certain aspects, a dsRNA molecule comprises a nucleic acid sequence complementary to about 100 to 1000 or about 200 to 1000 contiguous nucleotides of a target gene sequence. For example, in certain aspects, a dsRNA molecule comprises a nucleic acid sequence complementary to about 100 to 1000 or about 200 to 1000 contiguous nucleotides of the protein coding region of a target gene sequence. For example, in certain aspects, a dsRNA molecule comprises a nucleic acid sequence complementary to about 100 to 1000 or about 200 to 1000 contiguous nucleotides of the 5' UTR region or the 3' UTR region of a target gene sequence.

"Introducing" a nucleic acid is intended to mean presenting to the plant; or plant cell, a DNA or RNA construct. Processes for introducing polynucleotides into plants are known in the art including, and not limited to, stable transformation methods and transient transformation methods.

"Transformation" refers to the transfer of a nucleic acid fragment into the genome of a host organism, resulting in genetically stable inheritance. Host organisms containing the transformed nucleic acid fragments are referred to as "transgenic" organisms.

"Host cell" refers to the cell into which transformation of the DNA construct takes place and may include a yeast cell, a bacterial cell, and a plant cell. Examples of methods of plant transformation include Agrobacterium-mediated transformation (De Blaere et al., 1987, Meth. Enzymol. 143:277), particle-accelerated or "gene gun" transformation technology (Klein et al., 1987, Nature (London) 327:70-73; U.S. Patent No. 4,945,050) and protoplast transformation by electroporation or the use of chemicals such as PEG, among others.

"Stable transformation" is intended to mean that the polynucleotide introduced into a plant integrates into the genome of the plant and is capable of being inherited by the progeny thereof. "Transient transformation" or "transient expression" is intended to mean that the polynucleotide is introduced into the plant and does not integrate into the genome of the plant.

A "vector" is a construct that includes a replication system and sequences that are capable of transcription and translation of a polypeptide-encoding sequence in a given host cell. A number of vectors suitable for stable transfection of plant cells or for the establishment of transgenic plants have been described in, e.g., Pouwels et al., Cloning Vectors: A Laboratory Manual, 1985, supp. 1987; Weissbach and Weissbach, Methods for Plant Molecular Biology, Academic Press, 1989; and Flevin et al., Plant Molecular Biology Manual, Kluwer Academic Publishers, 1990. Typically, plant expression vectors include, for example, one or more cloned plant sequences under the transcriptional control of 5' and 3' regulatory sequences and a dominant selectable marker. 5' and 3' regulatory sequences comprise but are not limited to a promoter regulatory region (e.g., a regulatory region controlling inducible or constitutive, environmentally or developmentally regulated, or cell-or tissue-specific expression), transcription initiation start site, ribosome binding site, RNA processing signal, transcription termination site, and polyadenylation signal. In particular, the vectors according to the invention can be chosen from vectors pSB11 (*Ishida et al.,* (1996).), pBIOS898 (Biogemma, for sense construct), pBIOS895 (Biogemma, for RNAi construct), pBb7m34GW, pBb7m42GW7 (Karimi *et al.,* 2012), pDONR221 (Invitrogen).

By "plasmid", it is herein meant a double-stranded circular DNA. The plasmid may include a marker gene enabling to select the cells comprising said plasmid, an origin of replication to allow the cell to replicate the plasmid and/or a multiple cloning site allowing the insertion of a DNA fragment, in particular the polynucleotide according to the invention. In particular, said plasmid is chosen from L1457, L1465, L1478, L1482, L1479, L1483, L1542, L1543, L1540, L1541, as described in WO 2016/177887.

The vector preferably comprises an expression cassette of the polynucleotide of interest, i.e. a nucleic acid comprising to the polynucleotide of interest placed under the control of at least one expression signal allowing its expression.

The expression signal is particularly selected among a promoter, a terminator, an enhancer and their combinations. Suitable promoters, terminators and enhancers are well-known by the skilled person. In particular, the promoter may be a constitutive promoter selected in the group consisting of the rice actin promoter (Act1) and intron (McElroy et al., 1990, Plant Cell, 2 :163-171), the ubiquitin promoter of maize (Christensen et al., 1996, Transgenic. Res., 5 :213) or the CsVMV promoter (Verdaguer et al., 1998, Plant Mol Biol. 6:1129-39) and FAD₂ intron (patent application WO 2006/003186 from Biogemma). Other examples of constitutive promoters useful for expression include the 35S promoter, the 19S promoter (Kay et al., 1987, Science, 236:1299-1302), or the pCRV promoter (Depigny-This et al., 1992, Plant Molecular Biology, 20 :467-479). In a preferred embodiment, said construct is under the control of a constitutive promoter. In a most preferred embodiment, said construct is an RNAi construct, under the control of a constitutive promoter. Other suitable promoters could be used. It could be a tissue-specific promoter, for example a pollen-specific promoter, a promoter active in both anther and pollen, or an inducible promoter.

A terminator may be selected in the group consisting of the Nos terminator corresponding to the region in the non-coding 3' region of the nopaline synthase gene of the Ti-plasmid of *Agrobacterium tumefaciens* nopaline strain (Depicker et al. 1992) or the AtSac66 terminator. Others possible terminators are the polyA 35S terminator of the cauliflower mosaic virus (CaMV), described in the article of Franck *et al.,* (1980) or the histone terminator (EP 0 633 317).

Any other element like introns, enhancers, transit peptides, etc... may be comprised in the expression cassette. Introns and enhancers may be used to improve the expression of the polynucleotide of interest.

Suitable methods of introducing polynucleotides into plant cells are known by the man skilled in the art and include Agrobacterium- mediated transformation (U.S. Patent Nos. 5,563,055 and 5,981,840, and Ishida et al., (1996) High efficiency transformation of maize (Zea mays L.) mediated by Agrobacterium tumefaciens (Nat Biotechnol. 1996 Jun;14(6):745-50.), particle bombardment (Gordon-Kamm et al., 1990, Plant Cell 2:603-618) and electroporation of protoplasts (Rhodes et al., 1988, Science 240:204-207).

Methods are known in the art of the targeted insertion of a polynucleotide at a specific location in the plant genome. In one embodiment, the insertion of the polynucleotide at a desired genomic location is achieved using a site-specific recombination system. See, for example, WO99/25821, WO99/25854, WO99/25840, WO99/25855, and WO99/25853. Briefly, the polynucleotide according to the invention can be contained in a transfer cassette flanked by two non-identical recombination sites. An appropriate recombinase is needed for the integration of the transfer cassette fragment at the target site. Other methods without recombinase have also been described in WO 90/11354 but also in WO 96/14408. Said methods start from the observation that Double Strand Break (DSB) enhances the probability of homologous recombination at a given position. Said methods have been improved by the development of Meganucleases, Zn finger nuclease and other tools like TALENs and CRISPR/Cas9 system that have been developed to obtain targeted DSB.

The cells that have been transformed may be grown into plants in accordance with conventional ways. See, for example, McCormick et al. (1986) Plant Cell Reports 5:81-84.

The invention further relates to a genetically modified plant having increased resistance to a virus of the *Luteoviridae* family, in particular increased resistance to Barley Yellow Dwarf Virus (BYDV), which has been obtained by a method according to the invention.

In particular, said genetically modified plant comprises a mutated gene encoding a VHA-E protein, or an ortholog thereof, having disrupted interaction with the virus of the *Luteoviridae* family.

Depending on the ploidy of the plant to which resistance to a virus of the *Luteoviridae* family is to be conferred, the plant may comprise at least one mutated gene encoding VHA-E protein, or an ortholog thereof, having disrupted interaction with the virus of the *Luteoviridae* family. In particular, said genetically modified plant may comprise one, two or three mutated homeologous gene(s) encoding VHA-E protein having disrupted interaction (e.g. in barley, wheat, in particular common or durum wheat, maize, or rice), or encoding an ortholog of VHA-E protein having disrupted interaction (e.g. in a plant species other than barley, wheat, maize, and rice). In a preferred embodiment, the genetically modified plant is common wheat and comprises one, two or three mutated homeologous genes encoding a p.H160P vacuolar-type H⁺-ATPase subunit E (VHA-E) protein, at the heterozygous state or at the homozygous state.

In certain embodiments, the mutated (homeologous) gene(s) encoding the VHA-E protein, or an ortholog thereof, having disrupted interaction with the virus of the *Luteoviridae* family, is(are) at the heterozygous state or at the homozygous state.

In some embodiments the mutated gene encodes a p.H160P VHA-E protein, when the plant is barley, wheat, in particular common or durum wheat, maize, or rice, or an ortholog of p.H160P VHA-E protein when the plant is from a different plant species.

The invention also relates to a method for identifying mutant genes encoding vacuolar-type H⁺-ATPase subunit E (VHA-E) protein, or an ortholog thereof, conferring increased resistance to a virus of the *Luteoviridae* family to a plant susceptible to the virus of the *Luteoviridae* family, which comprises:
a) screening a genomic DNA library of said plant for identifying point mutation(s) in a gene encoding VHA-E protein, or an ortholog thereof;
b) generating genetically modified plants comprising a point mutation, preferably in a homozygous state, in a gene encoding VHA-E protein, or an ortholog thereof, as identified at step a);
c) characterizing if said genetically modified plants have increased resistance to the virus of the *Luteoviridae* family, or not, compared to plants susceptible to the virus of the *Luteoviridae* family comprising non mutated genes encoding VHA-E protein, or an ortholog thereof; and
d) identifying the mutant gene(s) encoding VHA-E protein, or an ortholog thereof, that are present in said genetically modified plants having increased resistance to the virus of the *Luteoviridae* family, as conferring increased resistance to the virus of the *Luteoviridae* family.

According to the method, the point mutation(s) in the gene encoding VHA-E protein, or an ortholog thereof are determined by comparison to the wild-type sequence of the encoded VHA-E protein, or ortholog thereof, in said plant.

According to an embodiment, at step b) genetically modified plants are generated that comprise a homozygous point mutation in a gene encoding VHA-E protein, or an ortholog thereof, as identified at step a).

The invention also relates to a method for identifying mutant plants having resistance to a virus of the *Luteoviridae* family, which comprises:
a) screening a plant library for identifying, in one or more plants of the library, point mutation(s) in a gene encoding VHA-E protein, or an ortholog thereof;
b) characterizing if said plants identified with point mutation(s) in a gene encoding VHA-E protein, or an ortholog thereof, have increased resistance to the virus of the *Luteoviridae* family, or not, compared to plants susceptible to the virus of the *Luteoviridae* family comprising non mutated genes encoding VHA-E protein, or an ortholog thereof.

According to this method, the plants constituting the plant library and the plants susceptible to the virus of the *Luteoviridae* family are of the same plant species.

According to an embodiment, the screening at step a) is performed by Targeting Induced Local Lesions IN Genomes (TILLING) or Ecotype TILLING (EcoTILLING).

According to some embodiments of the above methods, the plant susceptible to the virus of the *Luteoviridae* family is selected from the group consisting of barley, wheat, oat, rice, maize, or rapeseed.

According , to some embodiments of the above methods, the virus of the *Luteoviridae* family is BYDV.

The term "comprising," is inclusive or open-ended and does not exclude additional, unrecited elements and/or method steps. "Comprising" is a term of art that means that the named elements and/or steps are present, but that other elements and/or steps can be added and still fall within the scope of the relevant subject matter. Accordingly, in certain aspects, the disclosure of a method or product comprising elements and/or steps also discloses a method or product consisting of these elements and/or steps.

The term "consisting of" excludes any element, step, or ingredient not specifically recited.

The invention will be further illustrated in view of the following figures and examples.

### FIGURES

Figure 1. Description of the changes introduced into susceptible wheat TaVHA-E allele of chromosome 3A (TaVHA-E Chr3A) to confer resistance to BYDV. Sequences shown are SEQ ID NO: 45 to 48.
Figure 2. Alignment of wild-type VHA-E protein sequences from 1. rice (Oriza sativa), 2. common wheat *(Triticum aestivum L.),* 3-4. maize (Zea mays), and 5. barley *(Hordeum vulgare L.),* and 6. sequence of barley p.H160P VHA-E protein.
Figure 3: description of the plasmid used for functional validation of wheat and barley resistant variants by transgenesis.

### EXAMPLES

### Example 1. Reduction of the genomic interval containing the Ryd2 locus through fine mapping and the identification of the causal gene.

The Barley yellow dwarf virus (BYDV) resistant gene *Ryd2* (*Yd2*) has provided durable resistance in Barley *(Hordeum vulgare L.)* over the last 60 years to various BYDV isolates and has been the most extensively utilized BYDV resistance gene in Barley breeding programs. The semi-dominant gene *Yd2* was originally identified in Ethiopian spring barley landraces (Rasmusson and Schaller, 1959, Agron. J 51:661-664). The *Yd2* gene was identified close to the centromere on the long arm of Barley chromosome 3H (Collins et al. 1996, Theoret. Appl. Genetics 92, 858-864) and numerous genetic markers have been developed to track this gene, including the PCR-based markers on the *Ylp* gene (Ford et al. 1998, Mol. Breed. 4:23-31; Paltridge et al. 1998, Theor. Appl. Genet. 96:1170-1177).

The authors demonstrated complete linkage between gene *Yd2* and *Ylp,* which is logical since the locus sits in a region of low recombination. Despite complete linkage between *Yd2* and *Ylp* no homology was found between *Ylp* and any known plant disease resistance genes (Ford et al. 1998, Mol. Breed. 4:23-31,Jefferies *et* al. 2003, *122(1), 52-*56). Paltridge et al. (1998, Theor. Appl. Genet. 96:1170-1177) continued to work on the locus with a map-based approach using a population of 572 F2 individuals with the objective to clone the gene underlying the *Yd2* locus (King et al. 2002, A map-based approach towards cloning the Yd2 resistance gene in barley. In: Barley yellow dwarf disease: recent • advances and future strategies. CIMMYT, Mexico City: 82-92). However, due to low recombination rates of the region these authors failed to refine the genetic mapping resolution of the locus and the resistance mechanism or gene behind the *Ryd2* locus remains unknown.

We observed robust linkage of BYDV resistance and *Ylp* derived molecular markers (internal communication from Limagrain breeders). In 2019, we fine mapped the *Yd2* QTL interval using a large biparental population comprising 3720 F2 plants to alleviate the low recombination rate. The F2 population of 3720 F2 progeny consisted of two biparental cross derived from Limagrain's winter barley lines with and without the *Ryd2* QTL. 2037 F2 were derived from crossing LGBB18W169 (*Yd2+*) and LGBB18W144-74 (*Yd2-*), and 1290 F2 from the cross LGBB17W147 (*Yd2+*) by LGBB18W139D-32 (*Yd2-*).

We used a set of 46 SNP markers evenly distributed across the *Yd2* locus to identify and characterize recombinant plants. The recombinant plant screening identified 289 recombination events within the *Ryd2* QTL interval, which represented 25 different break points.

Given that the trait is semi-dominant it is possible to phenotype unfixed progenies and derive the phenotype of the F2 mother plant. The phenotypic characterization of the recombinants was organized in two batches. A first step to validate the mapping interval (2020) with F2 progenies and a second step concerning fixed recombinant plants within the interval of interest.

In 2020, F2 recombinant progenies were evaluated on two locations (France and Belgium) using BYDV natural infection in the field. This first batch of phenotypic data delimited the *Ryd2* QTL within the physical interval chr3H 448 226 200 bp to 471 964 721bp (23.74 Mb) on Morex IBSC V2 assembly (Masher et al. 2017, Nature 544, 427-433).

In 2021, fixed homozygous recombinant progenies within the 2020 mapping interval were phenotypically evaluated on two locations (France and Belgium) using BYDV natural infection in the field. We developed an additional 27 SNP markers to further characterize the position of the recombination breakpoints. The use of the new markers and new phenotyping data delimited the *Ryd2* QTL to within 5.837 Mb on the Morex IPK_V2 genome assembly between the physical positions chr3H:410336316 to chr3H:416209993. This region delineated by the markers chr3H_448184348 and contig_1567571_3217, contains 43 annotated genes (Morex IBSC_V2 and IPK_V2 gene prediction).

Functional annotation and homology to known plant disease resistance in the literature identified a subset of candidate genes within the area that could be involved with disease resistance (Table 1). From the 9 candidate genes described in Table 1, 4 candidate genes (Horvu_MOREX_3H01G349700, Horvu_MOREX_3H01 G349800, Horvu_MOREX_3H01G350400, Horvu_MOREX_3H01G351000) were selected as the most promising candidate genes according to the amount of evidence based on known domains or gene function associated to plant defense mechanisms (especially virus resistance in cereals and related species).

**Table1. The 9 candidate genes for Yd2**

| **IPK-V2_ref_gene** | **IBSC-V2_ref_gene** | **Gene function_IPK-V2** |
|---|---|---|
| Horvu_MOREX_3H01G 349700 | HORVU3Hr1G05 9290 | Ubiquitin conjugating enzyme E2 |
| Horvu_MOREX_3H01G 349800 | HORVU3Hr1G05 9280 | Vacuolar protein sorting protein 25 |
| Horvu_MOREX_3H01G 350400 | HORVU3Hr1G05 9320 | V-type proton ATPase subunit E |
| Horvu_MOREX_3H01G 351000 | HORVU3Hr1G05 9440 | Calcium-dependent lipid-binding (CaLB domain) family protein |
| Horvu_MOREX_3H01G 349500 | HORVU3Hr1G05 9250 | Ethylene-responsive transcription factor, putative |
| Horvu_MOREX_3H01G 352600 | HORVU3Hr1G05 9700 | AMSH-like ubiquitin thioesterase 1 |
| Horvu_MOREX_3H01G 351200 | HORVU3Hr1G05 9460 | F-box-like protein (Kelch repeat) |
| Horvu_MOREX_3H01G 351500 | HORVU3Hr1G05 9480 | Acidic endochitinase |
| Horvu_MOREX_3H01G 351600 | HORVU3Hr1G05 9470 | Pyruvate kinase |

The third stage of the cloning strategy involved the analysis of the nucleotide variations within the 43 annotated genes predicted under the QTL by sequencing a BYDV resistant line that carries *Ryd2* (LG-CAIMAN) and by performing a comparison of LG-CAIMAN to non-*Ryd2* lines in order to identify genetic differences in gene content or allelic diversity amongst candidate genes that could explain the resistance.

We sequenced and assembled LG-CAIMAN, a line carrying the *Ryd2* locus, using PacBio long read sequencing technology. The LG-CAIMAN assembly was generated by assembling 3 590 803 PacBio reads (14.8× sequence coverage) using an internal assembly pipeline. The whole QTL area (5.837 Mb on Morex IPK_V2 assembly) is located in the middle of a single LG-CAIMAN scaffold of 16.29 Mb.

The main objectives of this assembly were to compare gene content and structure to other well annotated non-*Ryd2* lines. A *de novo* gene annotation, using protein homology evidence and Morex IPKV2 predicted cDNA's, was performed on LG-CAIMAN whole genome assembly using an internal annotation pipeline. Resulting gene models were consolidated by projection of Morex_IPKV2, Morex_IBSCV2 and Wheat_IWGSC_V1 gene models using GMAP alignment program (Wu and Watanabe, 2005).

Structural differences in any of the 43 annotated genes or differences in gene content would provide extra evidence of one of these candidate genes being the causal gene underlying the resistance to BYDV. However, no major structural differences, nor presence or absence of genes were observed between LG-CAIMAN and Morex_IPK_V2 assembly. Therefore, we concluded that the causal polymorphism was not due to additional genes only found in LG-CAIMAN.

In search of more subtle functional differences in the causal gene we carried out a SNP & INDEL (insertion/deletion from 1 to 50 bp) variant calling using Freebayes (Garrison and Marth, 2012, Genomics, arXiv:1207.3907v2 in LG-CAIMAN, the tolerant line and 20 publicly available barley susceptible genomes (Jayakodi et al. 2020, Nature 588, 284-289). This variant calling identified 50 991 variants (SNP and INDEL) under the QTL area, 712 variants were specific to the tolerant line, including 16 within genes. However, only one candidate gene from Table 1 (Horvu_MOREX_3H01 G350400) contains a SNP specific to the tolerant line.

We enlarged this dataset with a new variant calling (SNPs and INDELS) on internal whole genome short read sequencing data from 31 barley lines including four BYDV tolerant lines carrying *Ryd2:* LG-CAIMAN, LG-ZEBRA, LG-ZODIAC, LG-BF19W037D-123 and 27 *non-Ryd2* lines. This variant calling identified 68 354 variants, 2 509 were specific to the *Ryd2* lines, including 8 variants within genes (shared with previous dataset). The LG-CAIMAN specific SNP detected in the candidate gene Horvu_MOREX_3H01 G350400 was reidentified and common to both datasets.

Analysis of variants in the gene promoter areas for both datasets (2500 bp upstream of the gene start), did not identify any *Ryd2* specific promoter variants that could modify the gene expression of the 43 annotated genes predicted under the QTL area. We then proceeded to annotate LG-CAIMAN specific variants in search of a functional significance. LG-CAIMAN specific variants were annotated using SnpEff (Cingolani et al. 2012, fly, 6(2), 80-92), and classified variants as high, moderate, low, or modifier-impact variants based on their potential impact at the protein level using the Morex_IPK_V2 gene annotation.

Table 2 summarizes the annotation of the 8 LG-CAIMAN specific variants shared across both datasets. 5 of the variants are intron variants on genes annotated as retrotransposons and were excluded from further analyses. The remaining 3 variants correspond to missense variants with putative moderate effects on the genes Horvu_MOREX_3H01G350300, Horvu_MQREX_3H01G350500 and Horvu_MOREX_3H01G350400. Of these 3 genes, only one (Horvu_MOREX_3H01G350400) has been associated with disease resistance in the literature.

**Table 2. The 8 LG-CAIMAN specific variants within genes with the SNEeff effect annotation**

| **CHR OM** | **POS IPK_V2** | **SNP_name** | **REF/ ALT** | **Gene IPK_V2** | **Gene_function IPK_V2** | **SNPeff ANN** | **SNPeff Impact** | **SNPeff aa_chan ge** |
|---|---|---|---|---|---|---|---|---|
| chr3 H | 410639 071 | LBV1_chr3H_4 10639071 | C/T | Horvu_MOREX_3 H01G349300 | filament-like protein (DUF869) | Intron variant | MODIF IER | no |
| chr3 H | 411282 033 | LBV1_chr3H_4 11282033 | A/G | Horvu_MOREX_3 H01G350200 | Retrotransposon protein | Intron variant | MODIF IER | no |
| chr3 H | 411282 306 | LBV1_chr3H_4 11282306 | T/C | Horvu_MOREX_3 H01G350200 | Retrotransposon protein | Intron variant | MODIF IER | no |
| chr3 H | 411283 592 | LBV1_chr3H_4 11283592 | A/C | Horvu_MOREX_3 H01G350200 | Retrotransposon protein | Intron variant | MODIF IER | no |
| chr3 H | 411286 507 | LBV1_chr3H_4 11286507 | C/A | Horvu_MOREX_3 H01G350200 | Retrotransposon protein | Intron variant | MODIF IER | no |
| chr3 H | 411286 619 | LBV1_chr3H_4 11286619 | C/T | Horvu_MOREX_3 H01G350200 | Retrotransposon protein | Intron variant | MODIF IER | no |
| chr3 H | 411544 488 | LBV1_chr3H_4 11544488 | C/T | Horvu_MOREX_3 H01G350300 | Potassium channel | Missense variant | MODE RATE | pos200:Gl y>Asp |
| **chr3 H** | **411660 796** | **LBV1_chr3H_ 411660796** | **A/C** | **Horvu_MOREX_3 H01G350400** | **V-type proton ATPase subunit E** | **Missense variant** | **MODE RATE** | **pos160:H is>Pro** |

We hypothesized that the Histidine to Proline change in the Horvu_MOREX_3H01G350400 gene was probably altering the interaction with the virus itself or with one or more components of the V-ATPase complex leading to BYDV tolerance. Understanding the nature of this interaction will enable the transfer of the resistance from barley to a related crop such as wheat. 3D structure of the protein for the LG-CAIMAN vs Morex alleles using missense3D (http://missense3d.bc.ic.ac.uk) indicates that the Histidine to Proline mutation does not result in any structural damage that could harm the integrity or functionality of the protein. However, it does change an external loop that could be involved in the interaction of the protein with the virus or other components of the complex. As other changes to this specific external domain of the protein could have an impact on the strength of the resistance mechanism, we actively mined for natural and induced variants in barley and wheat that could lead to new resistant sources in cereals. A list of induced and natural variants on both crops and their origin is described in Table 3. Mining natural variants on a large worldwide diversity panel of 1347 barley lines (IPK Gatersleben core 1000 accessions plus 347 breeding varieties, Milner et al. 2019, Nat. Genet. 51, 319-326) we identified 19 variants within the genomic sequence of VHA-E. As described in Table 3, most of the variants are non-functional (intronic). We note that the Histidine to Proline change on the Horvu_MOREX_3H01G350400 gene identified in previous diversity analyses is the only missense variant (causing an amino acid change on the protein) observed.

**Table 3: List of the VHA-E induced and natural variants in Hordeum vulgare and Triticum aestivum.**

| **Species** | **Variation origin** | **Variation type** | **Gene name** | **Genome reference** | **CHROM** | **Position** | **REF/ ALT** | **MAF** | **SNPeff ANN** | **SNPeff Impact** | **SNPeff aa_chang e** |
|---|---|---|---|---|---|---|---|---|---|---|---|
| *Hordeum vulgare* | Diversity panel | natural | Horvu_MOREX_3H0 1G350400 | Morex IPK_V2 | chr3H | 411658871 | C/T | 0.004 | intron_variant | MODIFIER | no |
| *Hordeum vulgare* | Diversity panel | natural | Horvu_MOREX_3H0 1G350400 | Morex IPK_V2 | chr3H | 411659026 | C/T | 0.023 | intron_variant | MODIFIER | no |
| *Hordeum vulgare* | Diversity panel | natural | Horvu_MOREX_3HO 1G350400 | Morex IPK_V2 | chr3H | 411659250 | C/T | 0.203 | intron_variant | MODIFIER | no |
| *Hordeum vulgare* | Diversity panel | natural | Horvu_MOREX_3H0 1G350400 | Morex IPK_V2 | chr3H | 411659509 | C/T | 0.478 | intron_variant | MODIFIER | no |
| *Hordeum vulgare* | Diversity panel | natural | Horvu_MOREX_3H0 1G350400 | Morex IPK_V2 | chr3H | 411659769 | C/A | 0.085 | intron_variant | MODIFIER | no |
| *Hordeum vulgare* | Diversity panel | natural | Horvu_MOREX_3H0 1G350400 | Morex IPK_V2 | chr3H | 411659770 | C/A | 0.087 | intron_variant | MODIFIER | no |
| *Hordeum vulgare* | Diversity panel | natural | Horvu_MOREX_3H0 1G350400 | Morex IPK_V2 | chr3H | 411659832 | A/G | 0.450 | intron_variant | MODIFIER | no |
| *Hordeum vulgare* | Diversity panel | natural | Horvu_MOREX_3H0 1G350400 | Morex IPK_V2 | chr3H | 411659866 | A/G | 0.346 | intron_variant | MODIFIER | no |
| *Hordeum vulgare* | Diversity panel | natural | Horvu_MOREX_3H0 1G350400 | Morex IPK_V2 | chr3H | 411659890 | G/T | 0.345 | intron_variant | MODIFIER | no |
| *Hordeum vulgare* | Diversity panel | natural | Horvu_MOREX_3H0 1G350400 | Morex IPK_V2 | chr3H | 411660309 | T/C | 0.344 | intron_variant | MODIFIER | no |
| *Hordeum vulgare* | Diversity panel | natural | Horvu_MOREX_3H0 1G350400 | Morex IPK_V2 | chr3H | 411660403 | G/A | 0.010 | intron_variant | MODIFIER | no |
| *Hordeum vulgare* | Diversity panel | natural | Horvu_MOREX_3H0 1G350400 | Morex IPK_V2 | chr3H | 411660614 | C/T | 0.015 | intron_variant | MODIFIER | no |
| *Hordeum vulgare* | Diversity panel | natural | Horvu_MOREX_3H0 1G350400 | Morex IPK_V2 | chr3H | 411660637 | C/T | 0.008 | intron_variant | MODIFIER | no |
| *Hordeum vulgare* | Diversity panel | natural | Horvu_MOREX_3H0 1G350400 | Morex IPK_V2 | chr3H | 411660796 | A/C | 0.015 | missense_varia nt | MODERATE | p.His160P ro |
| *Hordeum vulgare* | Diversity panel | natural | Horvu_MOREX_3H0 1G350400 | Morex IPK_V2 | chr3H | 411660980 | C/T | 0.022 | intron_variant | MODIFIER | no |
| *Hordeum vulgare* | Diversity panel | natural | Horvu_MOREX_3H0 1G350400 | Morex IPK_V2 | chr3H | 411661255 | C/G | 0.008 | intron_variant | MODIFIER | no |
| *Hordeum vulgare* | Diversity panel | natural | Horvu_MOREX_3HO 1G350400 | Morex IPK_V2 | chr3H | 411661557 | A/G | 0.005 | intron_variant | MODIFIER | no |
| *Hordeum vulgare* | Diversity panel | natural | Horvu_MOREX_3H0 1G350400 | Morex IPK_V2 | chr3H | 411661588 | C/T | 0.022 | intron_variant | MODIFIER | no |
| *Hordeum vulgare* | Diversity panel | natural | Horvu_MOREX_3H0 1G350400 | Morex IPK_V2 | chr3H | 411661704 | A/G | 0.009 | intron_variant | MODIFIER | no |
| *Hordeum vulgare* | Golden promise Tilling | EMS-induced | Horvu_GOLDEN_3H 01G318100 | Golden promise IPK_V1 | chr3H | 381683227 | C/T | - | missense_varia nt | MODERATE | p.Ser29Ph e |
| *Hordeum vulgare* | Golden promise Tilling | EMS-induced | Horvu_GOLDEN_3H 01G318100 | Golden promise IPK_V1 | chr3H | 381683236 | C/T | - | missense_varia nt | MODERATE | p.Ala32Va |
| *Hordeum vulgare* | Golden promise Tilling | EMS-induced | Horvu_GOLDEN_3H 01G318100 | Golden promise IPK_V1 | chr3H | 381685155 | G/A | - | missense_varia nt | MODERATE | p.Ser105A sn |
| *Hordeum vulgare* | Golden promise Tilling | EMS-induced | Horvu_GOLDEN_3H 01G318100 | Golden promise IPK_V1 | chr3H | 381685407 | G/A | - | missense_varia nt | MODERATE | p.Ala157T hr |
| *Hordeum vulgare* | Golden promise Tilling | EMS-induced | Horvu_GOLDEN_3H 01G318100 | Golden promise IPK_V1 | chr3H | 381685455 | C/T | - | missense_varia nt | MODERATE | p.Pro173S er |
| *Hordeum vulgare* | Golden promise Tilling | EMS-induced | Horvu_GOLDEN_3H 01G318100 | Golden promise IPK_V1 | chr3H | 381681816 | C/T | - | upstream_gene _variant | MODIFIER | no |
| *Hordeum vulgare* | Golden promise Tilling | EMS-induced | Horvu_GOLDEN_3H 01G318100 | Golden promise IPK_V1 | chr3H | 381681822 | C/T | - | upstream_gene _variant | MODIFIER | no |
| *Hordeum vulgare* | Golden promise Tilling | EMS-induced | Horvu_GOLDEN_3H 01G318100 | Golden promise IPK_V1 | chr3H | 381681992 | G/T | - | upstream_gene _variant | MODIFIER | no |
| *Hordeum vulgare* | Golden promise Tilling | EMS-induced | Horvu_GOLDEN_3H 01G318100 | Golden promise IPK_V1 | chr3H | 381682149 | C/T | - | upstream_gene _variant | MODIFIER | no |
| *Hordeum vulgare* | Golden promise Tilling | EMS-induced | Horvu_GOLDEN_3H 01G318100 | Golden promise IPK_V1 | chr3H | 381682220 | A/T | - | upstream_gene _variant | MODIFIER | no |
| *Hordeum vulgare* | Golden promise Tilling | EMSinduced | Horvu_GOLDEN_3H 01G318100 | Golden promise IPK_V1 | chr3H | 381684186 | A/T | - | splice_acceptor _variant | HIGH | no |
| *Hordeum vulgare* | Golden promise Tilling | EMS-induced | Horvu_GOLDEN_3H 01G318100 | Golden promise IPK_V1 | chr3H | 381684245 | G/A | - | missense_varia nt | MODERATE | p.Glu54Ly s |
| *Hordeum vulgare* | Golden promise Tilling | EMS-induced | Horvu_GOLDEN_3H 01G318100 | Golden promise IPK_V1 | chr3H | 381685190 | C/T | - | missense_varia nt | MODERATE | p.Leu117P he |
| *Hordeum vulgare* | Golden promise Tilling | EMS-induced | Horvu_GOLDEN_3H 01G318100 | Golden promise IPK_V1 | chr3H | 381685726 | G/A | - | missense_varia nt | MODERATE | p.Gly189S er |
| *Hordeum vulgare* | Golden promise Tilling | EMS-induced | Horvu_GOLDEN_3H 01G318100 | Golden promise IPK_V1 | chr3H | 381686451 | G/A | - | splice_acceptor _variant | HIGH | no |
| *Hordeum vulgare* | Golden promise Tilling | EMS-induced | Horvu_GOLDEN_3H 01G318100 | Golden promise IPK_V1 | chr3H | 381686456 | G/A | - | missense_varia nt | MODERATE | p.Arg221 Gln |
| *Hordeum vulgare* | Golden promise Tilling | EMS-induced | Horvu_GOLDEN_3H 01G318100 | Golden promise IPK_V1 | chr3H | 381683175 | C/T | - | stop_gained | HIGH | p.Gln12* |
| *Triticum aestivum* | Diversity panel | natural | TraesCS3A01G2387 00 | CS_IWGSC_V2 | chr3A | 446475383 | G/T | 0.031 | intron_variant | MODIFIER | |
| *Triticum aestivum* | Diversity panel | natural | TraesC53A01G2387 00 | CS_IWGSC_V2 | chr3A | 446476742 | G/A | 0.002 | intron_variant | MODIFIER | |
| *Triticum aestivum* | Diversity panel | natural | TraesC53A01G2387 00 | CS_IWGSC_V2 | chr3A | 446477134 | C/G | 0.001 | missense_varia nt | MODERATE | p.lle79Me t |
| *Triticum aestivum* | Diversity panel | natural | TraesC53A01G2387 00 | CS_IWGSC_V2 | chr3A | 446477310 | G/T | 0.001 | intron_variant | MODIFIER | |
| *Triticum aestivum* | Diversity panel | natural | TraesCS3A01G2387 00 | CS_IWGSC_V2 | chr3A | 446477527 | C/G | 0.053 | missense_varia nt | MODERATE | p.His178A sp |
| *Triticum aestivum* | Diversity panel | natural | TraesCS3A01G2387 00 | CS_IWGSC_V2 | chr3A | 446477603 | C/A | 0.000 | intron_variant | MODIFIER | |
| *Triticum aestivum* | Diversity panel | natural | TraesCS3A01G2387 00 | CS_IWGSC_V2 | chr3A | 446479211 | G/T | 0.001 | missense_varia nt | MODERATE | p.Arg214L eu |
| *Triticum aestivum* | Diversity panel | natural | TraesC53A01G2387 00 | CS_IWGSC_V2 | chr3A | 446479232 | G/A | 0.003 | splice_region_v ariant | LOW | |
| *Triticum aestivum* | Diversity panel | natural | TraesCS3A01G2387 00 | CS_IWGSC_V2 | chr3A | 446479640 | C/T | 0.002 | intron_variant | MODIFIER | |
| *Triticum aestivum* | Diversity panel | natural | TraesC53A01G2387 00 | CS_IWGSC_V2 | chr3A | 446479683 | C/A | 0.001 | intron_variant | MODIFIER | |
| *Triticum aestivum* | Diversity panel | natural | TraesCS3B01G2674 00 | CS_IWGSC_V2 | chr3B | 429272730 | C/T | 0.259 | intron_variant | MODIFIER | |
| *Triticum aestivum* | Diversity panel | natural | TraesCS3B01G2674 00 | CS_IWGSC_V2 | chr3B | 429273518 | C/G | 0.007 | intron_variant | MODIFIER | |
| *Triticum aestivum* | Diversity panel | natural | TraesCS3B01G2674 00 | CS_IWGSC_V2 | chr3B | 429275154 | T/C | 0.035 | intron_variant | MODIFIER | |
| *Triticum aestivum* | Diversity panel | natural | TraesCS3D01G2391 00 | CS_IWGSC_V2 | chr3D | 330637811 | G/T | 0.001 | missense_varia nt | MODERATE | p.Gln12Hi s |
| *Triticum aestivum* | Diversity panel | natural | TraesCS3D01G2391 00 | CS_IWGSC_V2 | chr3D | 330637926 | T/C | 0.124 | intron_variant | MODIFIER | |
| *Triticum aestivum* | Diversity panel | natural | TraesCS3D01G2391 00 | CS_IWGSC_V2 | chr3D | 330637943 | A/G | 0.152 | intron_variant | MODIFIER | |
| *Triticum aestivum* | Diversity panel | natural | TraesCS3D01G2391 00 | CS_IWGSC_V2 | chr3D | 330638003 | T/C | 0.170 | intron_variant | MODIFIER | |
| *Triticum aestivum* | Diversity panel | natural | TraesCS3D01G2391 00 | CS_IWGSC_V2 | chr3D | 330638014 | C/T | 0.137 | intron_variant | MODIFIER | |
| *Triticum aestivum* | Diversity panel | natural | TraesCS3D01G2391 00 | CS_IWGSC_V2 | chr3D | 330638065 | G/A | 0.003 | intron_variant | MODIFIER | |
| *Triticum aestivum* | Diversity panel | natural | TraesCS3D01G2391 00 | CS_IWGSC_V2 | chr3D | 330640521 | C/A | 0.001 | missense_varia nt | MODERATE | p.His108A sn |
| *Triticum aestivum* | Diversity panel | natural | TraesCS3D01G2391 00 | CS_IWGSC_V2 | chr3D | 330640822 | C/T | 0.001 | missense_varia nt | MODERATE | p.Ser175P he |
| *Triticum aestivum* | Diversity panel | natural | TraesCS3D01G2391 00 | CS_IWGSC_V2 | chr3D | 330640966 | A/T | 0.023 | intron_variant | MODIFIER | |
| *Triticum aestivum* | Diversity panel | natural | TraesCS3D01G2391 00 | CS_IWGSC_V2 | chr3D | 330641193 | C/A | 0.002 | intron_variant | MODIFIER | |
| *Triticum aestivum* | Diversity panel | natural | TraesCS3D01G2391 00 | CS_IWGSC_V2 | chr3D | 330641358 | A/G | 0.044 | intron_variant | MODIFIER | |
| *Triticum aestivum* | Diversity panel | natural | TraesCS3D01G2391 00 | CS_IWGSC_V2 | chr3D | 330641378 | G/A | 0.001 | intron_variant | MODIFIER | |
| *Triticum aestivum* | Diversity panel | natural | TraesCS3D01G2391 00 | CS_IWGSC_V2 | chr3D | 330641388 | C/A | 0.001 | intron_variant | MODIFIER | |
| *Triticum aestivum* | Diversity panel | natural | TraesCS3D01G2391 00 | CS_IWGSC_V2 | chr3D | 330641654 | A/G | 0.004 | intron_variant | MODIFIER | |
| *Triticum aestivum* | Cadenza Tilling | EMS-induced | TraesC53A02G2387 00 | CS_IWGSC_V2 | chr3A | 446477449 | G/A | - | missense variant | MODERATE | p.Glu152L ys |
| *Triticum aestivum* | Cadenza Tilling | EMS-induced | TraesCS3A02G2387 00 | CS_IWGSC_V2 | chr3A | 446477525 | G/A | - | missense variant | MODERATE | p.Ser177A sn |
| *Triticum aestivum* | Cadenza Tilling | EMS-induced | TraesCS3B02G2674 00 | CS_IWGSC_V2 | chr3B | 429273302 | C/T | - | stop gained | HIGH | p.Gln61* |
| *Triticum aestivum* | Cadenza Tilling | EMS-induced | TraesCS3B02G2674 00 | CS_IWGSC_V2 | chr3B | 429274451 | G/A | - | missense variant | MODERATE | p.Glu161L ys |
| *Triticum aestivum* | Cadenza Tilling | EMS-induced | TraesC53B02G2674 00 | CS_IWGSC_V2 | chr3B | 429274511 | G/A | - | missense variant | MODERATE | p.Glu181L ys |
| *Triticum aestivum* | Cadenza Tilling | EMS-induced | TraesCS3B02G2674 00 | CS_IWGSC_V2 | chr3B | 429274514 | C/T | - | missense variant | MODERATE | p.His182T yr |

### Example 2. Use of Horvu MOREX 3H01 G350400 LG-CAIMAN SNP as a Yd2 diagnostic marker.

The SNP variant A/C at position chr3H:411660796 in the gene Horvu_MOREX_3H01G350400 can be followed by the KASPar SNP marker named LBV1_chr3H_411660796 and used as a perfect diagnostic marker to follow the causal polymorphism and allow selection of *Yd2* tolerant lines in breeding programs (see marker context sequence in Table 4 and ASP primer sequences in Table 5).

**Table 4: LBV1 chr3H 411660796 marker sequences showing a single nucleotide polymorphism (SNP) between tolerant and susceptible barley genomes**

| **Marker sequence of resistance allele** | **Marker sequence of susceptible allele** |
|---|---|
| SEQ ID NO: 22 | SEQ ID NO: 23 |
| AAAGAAGATCACCATAATGTGGAATCTGTA CTGCATTCAGCAAAGAATGAATATGCATCA AAAGCAGATGTCC[C]TGAGCCAGAGATAC TAGTTGACCATAGTGTCTACCTGCCACCTT CTCCGAGCCATGATGATAAGCACGGCCAG ATTTGCCATGGAGGTGTTGTGCTGGCTTCT CGA | AAAGAAGATCACCATAATGTGGAATCTGTA CTGCATTCAGCAAAGAATGAATATGCATCA AAAGCAGATGTCC[A]TGAGCCAGAGATAC TAGTTGACCATAGTGTCTACCTGCCACCTT CTCCGAGCCATGATGATAAGCACGGCCAG ATTTGCCATGGAGGTGTTGTGCTGGCTTCT CGA |

**Table 5: KASPar primers for use in detecting LBV1 chr3H 411660796 SNP variant**

| **SEQ ID NO:** | **MARKER NAME** | **Allele** | **Sequence** |
|---|---|---|---|
| 24 | LBV1_chr3H _411660796 Allele X | Susceptible | GAAGGTCGGAGTCAACGGATTGGTCAACTAGTATCTC TGGCTCAT |
| 25 | LBV1_chr3H _411660796 Allele Y | Resistant | GAAGGTGACCAAGTTCATGCTGTCAACTAGTATCTCTG GCTCAG |
| 26 | LBV1_chr3H _411660796 Common | | TACTGCATTCAGCAAAGAATGAATATGCAT |

The SNP LBV1_chr3H_411660796 defined by the inventors as the *Yd2* causal variant, is the same variant as the PCR-based markers *Ylp* described by Ford et al. (1998, Mol. Breed. 4:23-31) and Paltridge et al. (1998, Theor. Appl. Genet. 96:1170-1177). This variant was described by the authors as being closely linked to a gene encoding a subunit E of vacuolar proton translocating ATPase (Ford et al. 1998, Mol. Breed. 4:23-31), but was in fact the causal variant identified serendipitously via two-dimensional SDS-PAGE protein gel electrophoresis (Holloway et al. 1992, Theor Appl Genet 85:346-352).

### Example 3: Identification of V-type proton ATPase subunit E (VHA-E) gene in wheat, maize and rice

Identification of *VHA-E* gene in wheat, maize and rice genes was done by Blast analysis using the barley VHA-E protein sequence as bait (Horvu_MOREX_3H01G350400). For wheat, 3 homeolog sequences were found TraesCS3A01G238700/TraesCS3B02G267400/TraesCS3D02G239100), 2 for maize (Zm00001d043991/ Zm00001 d01 1504) and one for rice (Os01g0659200/ LOC_Os01g46980.1).

The sequence of these genes or corresponding CDS are disclosed as SEQ ID NO: 1 (barley), SEQ ID NO: 4, 6 and 8 (wheat), SEQ ID NO: 14 and 15 (maize) and SEQ ID NO: 10, as detailed previously in the description.

Based on the result in Barley, these orthologues are good candidates to confer virus resistance in the corresponding crops as wheat, maize or rice.

### Example 4: Yeast Two-Hybrid (Y2H) results to identify interaction between VHA-E protein and BYDV viral protein

For the Y2H assay, the activation domain (AD) vector pGADT7 and the GAL4 DNA-binding domain (BD) vector pGBKT7 were used (Matchmaker Gold Yeast Two-Hybrid System (Clontech, Mountain View, CA, USA). The prey vector pGADT7 (activation domain (AD) with VHA-E) and bait vector pGBKT7 (binding domain (BD) with the 6 BYDV proteins (MP, RTD, VP1, VP2, CP and VP6) (independently) harboring the genes to be tested were co-transformed pairwise into the yeast (*Saccharomyces cerevisiae*) strains Y187 & Y2HGold.

Y2H based on GAL4 was done to identify the interaction between BYDV proteins (MP, RTD, VP1, VP2, CP and VP6) with the VHA-E protein (wheat and barley) homologs. The bait vectors pGBKT7-BYDV_RTD, pGBKT7-BYDV_MP, pGBKT7-BYDV_VP1, pGBKT7-BYDV_VP2, pGBKT7-BYDV_VP6, and pGBKT7-BYDV_CP were individually co-transformed with the prey vectors pGADT7-TaVHA-E (wheat protein sequence), pGADT7-HvVHA-E (barley protein sequence) into the *Saccharomyces cerevisiae* strains Y187 & Y2HGold.

As a result, the yeast cells co-transformed with pGBKT7-BYDV_RTD and pGBKT7-BYDV_MP and pGADT7-TaVHA-E or pGADT7-HvVHA-E grow well which indicate an interaction between the 2 BYDV proteins MP and RTD and VHA-E proteins (from wheat and barley), whereas with pGBKT7-BYDV_VP1, pGBKT7-BYDV_VP2, pGBKT7-BYDV_VP6, and pGBKT7-BYDV_CP no yeast cells growing were identified, indicating no interaction between VP1, VP2, CP and VP6 BYDV proteins and VHA-E proteins. In conclusion, the Y2H assay demonstrated that out of the nine candidate genes (Table 1) underlying the *Ryd2* QTL, only VHA-E interacts with viral proteins, again suggesting that VHA-E is the causal gene.

### Example 5: Transgenic expression of VHA-E variants in wheat fielder genotype

Functional validation of the wheat and barley resistant variants (TaVHA-E_H160P) and (HvVHA-E_H160P) was done by transgenesis by expressing resistant variants in a susceptible background. The rationale for this experiment is based on the inability of the virus to infect plants with a high expression of a resistant (variant) allele of VHA-E (e.g. *Ta*VHA-E_H160P) through a so-called titration effect. Indeed, the abundance of the resistant allele will strongly impact the capacity of the viral protein to use the native susceptible allele, which will be in much lower quantity than the resistant allele.

An over-representation of resistant VHA-E_H160P variants from wheat and barley under the control of the *Zm*Ubi promoter was produced in wheat to confer tolerance to the virus by titration of the wild-type allele.

A set of 3 different constructs has been used to validate the VHA-E_H160P variant:
- 7a_VHA-E_S: wheat susceptible protein sequence (as a control)
- Ta VHA-E_R: wheat resistant protein sequence (H160P)
- *Hv_*VHA-E_Caiman : barley resistant protein sequence (H160P)

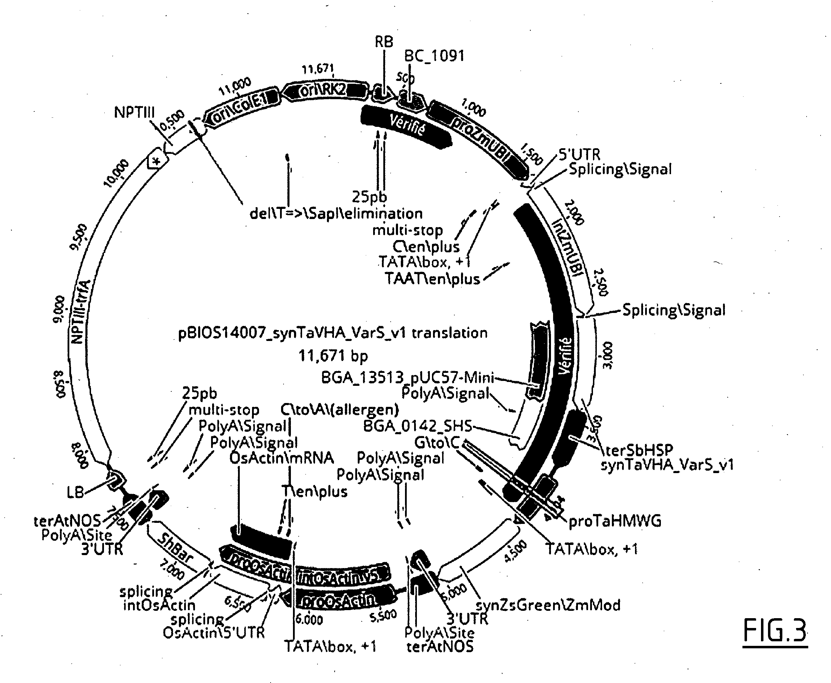

A circular map of the plasmid used for transgenesis is shown in Figure 3.

### Example 6: Production of VHA-E resistant variants (H160P) for BYDV resistance in wheat by Prime Editing method (SDN2)

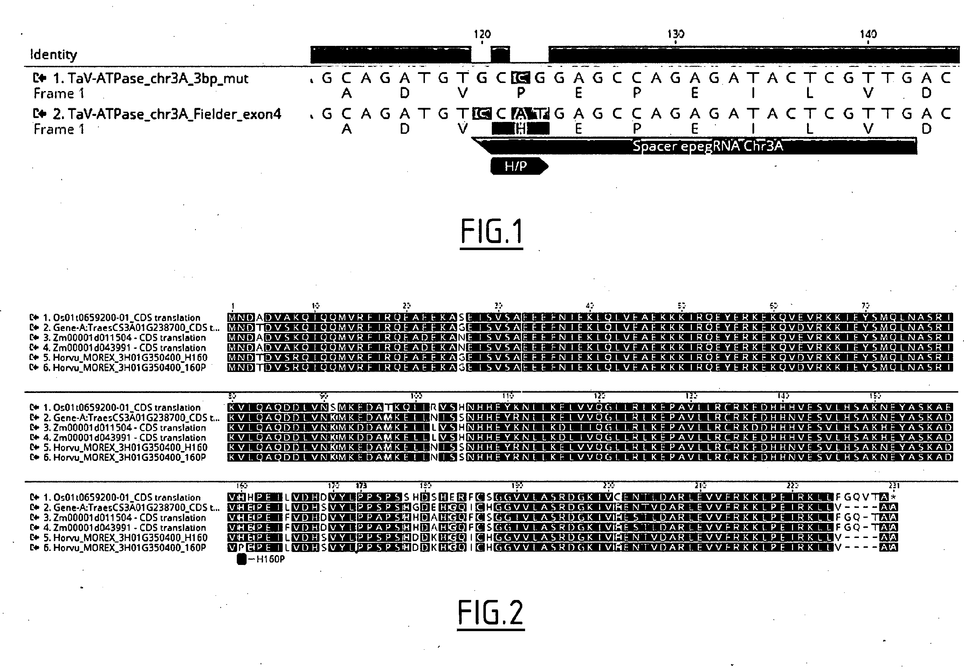

The goal of this experiment is to change by genome editing a susceptible wheat TaVHA-E allele into a resistant one. The nucleotide required to generate the modification of the amino-acid in position 160, replacing a histidine to a proline (H160P) was achieved by prime editing methodology (Anzalone et al., 2019, Nature, 576, pages149-157) using epegRNAs (Nelson et al., 2021, Nat Biotechnol., 40(3):402-410). These epegRNAs had a. 3' tevoprecQ1 pseudo-knot sequence designed to stabilize the pegRNA (Nelson et al., 2021, Nat Biotechnol., 40(3):402-410). The Reverse Template region of the epegs were designed to introduce three edits, two to change His to Pro (C**AT** to C**CG**) and one silent edit Val GT**C** to GT**G**) (See Fig. 1 for changes introduced to TaVHA-E Chr3A). Silent edits were introduced to improve efficiency of editing (Chen et al, 2021, Cell 184, 5635-5652). Given the sequence variation between each genome a specific epegRNA was designed to target and edit each genome (SEQ ID NO: 27-34 and table 6). All 3 epegRNAs (TaU6-TaVHA_Chr3A_epegRNA_v1: SEQ ID NO: 36, TaU6-TaVHA_Chr3B_epegRNA_v1: SEQ ID NO: 37, TaU6-TaVHA_Chr3D_epegRNA_v1: SEQ ID NO: 38), each driven by a wheat U6 promoter (SEQ ID NO: 35), were cloned together with a ZmUbi-nickaseCas9(H840A)::MMLV-term ZmHSP cassette (SEQ ID NO: 39) into a binary vector for plant transformation. The plant binary vector contained a Rice Actin promoter-Bar cassette for the section of transformants on the herbicide BASTA. The binary vector was transferred into agrobacterium and the susceptible wheat variety Fielder was transformed using a standard wheat transformation protocol. TaVHA-E prime-edited plants identified by Next Generation Sequence (NGS) analysis of DNA from leaf samples or primary transformants. Progeny of edited plants with the desired nucleotide change on each 3 homeologs alone or in combination were produced and phenotyped for resistance to BYDV.

**Table 6: epegRNA designs to target and edit susceptible alleles in each genome**

| **epegRNA** | **Spacer** | **RT template** | **PBS** |
|---|---|---|---|
| TaVHA_Chr3A_v1 SEQ ID NO: 42 | Caacgagtatctctggctca SEQ ID NO: 27 | aaagcagatgtGCcGga SEQ ID NO: 28- | Gccagagata SEQ ID NO: 29 |
| TaVHA_Chr3B_v1 SEQ ID NO: 43 | Caactagtatctcgggctca SEQ ID NO: 30 | aaagcagatgtGCcGga SEQ ID NO: 31 | Gcccgagat |
| TaVHA_Chr3D_v1 SEQ ID NO:44 | Caactagtatctctggctca SEQ ID NO: 32 | aaagcagatgtGCcGga SEQ ID NO: 33 | Gccagagata SEQ ID NO: 34 |

## Claims

1. A method for providing a plant having increased resistance to a virus of the *Luteoviridae* family, which comprises modifying the plant so that it is able to express a vacuolar-type H⁺-ATPase subunit E (VHA-E), or an ortholog thereof, having disrupted interaction with the virus of the *Luteoviridae* family.

2. The method according to claim 1, wherein the plant is barley *(Hordeum vulgare L.*)*,* common wheat (*Triticum aestivum L.*)*,* durum wheat (*Triticum durum* or *Triticum turgidum* subsp. *Durum),* maize (*Zea mays)*, or rice I*(Oryza sativa*)*.*

3. The method according to claim 1 or 2, which comprises mutating a gene encoding VHA-E, or an ortholog thereof, in the genome of the plant to encode a VHA-E protein, or an ortholog thereof, having disrupted interaction with the virus of the *Luteoviridae* family.

4. The method according to any one of claims 1 to 3, wherein the method comprises mutating the gene encoding VHA-E in a codon encoding Histidine at position 160 of the VHA-E protein when the plant is barley, common or durum wheat, maize, or rice, or, in a different plant species, mutating an ortholog of the gene encoding VHA-E protein in a codon homologous to the one encoding Histidine at position 160 of VHA-E protein of barley, common wheat, maize, or rice .

5. The method, according to any one of claims 1 to 4, wherein the method comprises mutating the gene encoding VHA-E protein to encode a p.H160P VHA-E protein when the plant is barley, common or durum wheat, maize, or rice.

6. The method according to claim 1 or 2, which comprises introducing into the plant a nucleic acid encoding a VHA-E protein, or an ortholog thereof, having disrupted interaction with the virus of the *Luteoviridae* family.

7. The method according to claim 6, which comprises introducing into the plant a nucleic acid encoding a p.H160P VHA-E protein when the plant is barley, common or durum wheat, maize, or rice.

8. A method for providing a plant having increased resistance to a virus of the *Luteoviridae* family, which comprises mutating a gene encoding vacuolar-type H+-ATPase subunit E (VHA-E) in the genome of the plant to encode a p.H160P VHA-E protein, wherein the plant is barley (*Hordeum vulgare L.*)*,* common wheat (*Triticum aestivum L.*)*,* durum wheat (*Triticum durum* or *Triticum turgidum* subsp. *Durum*)*,* maize (Zea mays), or rice (*Oryza sativa*)*,* or to encode an ortholog of p.H160P VHA-E protein when the plant is from a different plant species.

9. The method according to any one of claims 3 to 5 and 8, wherein the plant is common wheat, and the method comprises mutating one, two or three homeologous genes encoding VHA-E.

10. The method according to any one of claims 3 to 5 and 8 to 9, wherein mutating the gene encoding VHAE is performed by a gene editing method.

11. A method for providing a plant having increased resistance to a virus of the *Luteoviridae* family, which comprises inhibiting expression of vacuolar-type ^{H+}-ATPase subunit E (VHA-E) protein, or an ortholog thereof, in the plant.

12. The method according to any one of claims 1 to 11, wherein the virus of the *Luteoviridae* family is a Barley Yellow Dwarf Virus (BYDV).

13. A genetically modified plant having increased resistance to a virus of the *Luteoviridae* family, which has been obtained by a method according to any one of claims 1-12.

14. The genetically modified plant according to claim 13, which comprises a mutated gene encoding a p.H160P vacuolar-type H⁺-ATPase subunit E (VHA-E ) protein, when the plant is barley (*Hordeum vulgare L.*)*,* common wheat (*Triticum aestivum L.*)*,* durum wheat (*Triticum durum* or *Triticum turgidum* subsp. *Durum*)*,* maize (*Zea mays)*, or rice (*Oryza sativa*) or an ortholog of p.H160P VHA-E protein when the plant is from a different plant species.

15. The genetically modified plant according to claim 13 or 14, wherein the plant is common wheat and comprises one, two or three mutated homeologous genes encoding a p.H160P vacuolar-type H⁺-ATPase subunit E (VHA-E) protein.

16. A method for identifying mutant genes encoding vacuolar-type H⁺-ATPase subunit E (VHA-E) protein, or an ortholog thereof, conferring increased resistance to a virus of the *Luteoviridae* family to a plant susceptible to the virus of the *Luteoviridae* family, which comprises:
a) screening a genomic DNA library of said plant for identifying point mutation(s) in a gene encoding VHA-E protein, or an ortholog thereof;
b) generating genetically modified plants comprising a point mutation in a gene encoding VHA-E protein, or an ortholog thereof, as identified at step a);
c) characterizing if said genetically modified plants have increased resistance to the virus of the *Luteoviridae* family, or not, compared to plants susceptible to the virus of the *Luteoviridae* family comprising non mutated genes encoding VHA-E protein, or an ortholog thereof,; and
d) identifying the mutant gene(s) encoding VHA-E protein, or an ortholog thereof, that are present in said genetically modified plants having increased resistance to the virus of the *Luteoviridae* family, as conferring increased resistance to the virus of the *Luteoviridae* family.

17. A method for identifying mutant plants having resistance to a. virus of the *Luteoviridae* family, which comprises:
c) screening a plant library for identifying, in one or more plants of the library, point, mutation(s) in a gene encoding VHA-E protein, or an ortholog thereof;
d) characterizing if said plants identified with point mutation(s) in a gene encoding VHA-E protein, or an ortholog thereof, have increased resistance to the virus of the *Luteoviridae* family, or not, compared to plants susceptible to the virus of the *Luteoviridae* family comprising non mutated genes encoding VHA-E protein, or an ortholog thereof.
